# EUROPÄISCHE PATENTSCHRIFT

(11) **EP 0 384 122 B1**
(45) Veröffentlichungstag und Bekanntmachung des Hinweises auf die Patenterteilung: **22.09.1999**
(21) Anmeldenummer: 90101045.4
(22) Anmeldetag: 19.01.1990
(51) Int. Cl.: C12N 15/15, C12P 21/02, A61K 38/55

(54) **Mutanten von humanem Antithrombin III**
Mutants of human antithrombin III
Mutants de l'anti-thrombine III humaine

(30) Priorität: 24.01.1989 DE 3901917
(43) Veröffentlichungstag der Anmeldung: 29.08.1990
(73) Patentinhaber: Centeon Pharma GmbH, 35002 Marburg (DE)
(72) Erfinder: Zettlmeissl, Gerd, Dr., D-3551 Lahntal (DE); Karges, Hermann Erich, Dr., D-3550 Marburg (DE); Becker, Achim, D-3563 Dautphetal (DE)

(56) Entgegenhaltungen:
- EP-A- 0 155 188
- EP-A- 0 256 302
- EP-A- 0 262 805
- WO-A-91/00291
- BIOTECHNOLOGY, Band 5, Nr. 7, Juli 1987, Seiten 720-725, New York, US; G. ZETTMEISSL et al.: "Expression of biologically active human antithrombin III in chinese hamster ovary cells"
- PROTEIN ENGINEERING, Band 1, Nr. 1, 1986, Seiten 29-35, Eynsham, GB; S. JALLAT et al.: "Altered specificities of genetically engineered alpha1 antitrypsin variants"
- THE JOURNAL OF BIOLOGICAL CHEMISTRY, Band 263, Nr. 31, 5. November 1988, Seiten 15849-15852, The American Society for Biochemistry and Molecular Biology, Inc., US; A.W. STEPHENS et al.: "Site-directed mutagenesis of the ractive center (Serine 394) of antithrombin III"
- BLOOD, Band 72, Nr. 5, November 1988, Seiten 1817-1821, New York, NY, US; S.L. THEIN et al.: "Use of synthetic oligonucleotides in the characterization of antithrombin III Northwick Park (393 CGT - TGT) and antithrombin III Glasgow (393 CGT - CAT)"

## Beschreibung

Die Erfindung betrifft Mutanten von AT III, die gegenüber Wildtyp AT III verbesserte Eigenschaften besitzen. Die Veränderung an einer oder mehreren potentiellen Glykosylierungsstellen (z.B. Asn 135, Asn 155) erhöht die Heparinbindungs-/Heparinaktivierungseigenschaften unter Beibehaltung der Proteasespezifität von AT III. Änderungen an Pos. Arg 393 führen zu Änderungen der Spezifität gegenüber Proteasen. Die Kombination der Mutationen bewirkt in der Regel eine Potenzierung der Verbesserungen.

Die für humanes Antithrombin III (AT III) kodierende cDNA und deren Expression in E.coli sind in der europäischen Patentanmeldung EP 0 090 505 A2 beschrieben. Darüberhinaus ist die Expression von AT III in rekombinanten Hefen (EP 0 256 302 A2) und Säugerzellen (DE 3 624 453 Al) gezeigt. Bei diesen Experimenten stellte sich heraus, daß nur AT III, welches von Säugerzellen ins Kulturmedium sezerniert wird, vollständige biologische Aktivität in vitro zeigt und eine dem Plasmaprotein sehr ähnliche komplexe Kohlehydratstruktur besitzt (Zettlmeißl et al., BioTechnology, 1987, 5, 720-725).

Das Molekulargewicht von rekombinantem AT III aus Säugerzellen entspricht mit ca. 58 kd dem des aus Plasma gereinigten Proteins. Die Aminosäuresequenz von reifem humanem AT III ist in Tab. 1 dargestellt.

AT III ist ein Mitglied der Proteinfamilie der Serpine und besitzt demnach hohe Homologie zu Proteaseinhibitoren wie Alpha-1-Antitrypsin, Alpha-2-Antiplasmin, Heparinkofaktor II, Alpha-1-Antichymotrypsin, Plasminogenaktivator inhibitor, etc. Wenn die Serinprotease Thrombin mit AT III interagiert, spaltet sie die Arg393-Ser394 Bindung und es kommt zur Ausbildung eines kovalenten AT III-Thrombin-Komplexes. Mit der Komplexbildung verliert Thrombin seine Proteaseaktivität. In Abwesenheit von Heparin ist AT III ein relativ schlechter Inhibitor von Thrombin. Durch optimale Konzentrationen von Heparin wird die Geschwindigkeitskonstante der AT III-Thrombinassoziationsreaktion um mindestens Faktor 2000 gesteigert (Hoylaerts et al., J. Biol. Chem., 1984, 259, 5670-5677). Im menschlichen Plasma existieren zwei Formen von AT III (alpha und beta) mit unterschiedlicher Heparinaffinität (Peterson und Blackburn, J. Biol. Chem., 1985, 260, 610-615; Brennan et al., FEBS LETT., 1987, 219, 431-436). Während AT IIIalpha, das zur 90-95% im Plasma vorkommt, an den Asn-Resten 96, 135,155 und 192 Kohlehydratseitenketten trägt, sind bei AT IIIbeta nur die Stellen 96, 155 und 192 besetzt. Die physiologische Rolle der beiden AT III-Formen ist nicht bekannt.

Die Technik der gezielten Mutagenese erlaubt es, spezifische Änderungen in die AT III cDNA einzubringen, die zu Veränderungen in der Aminosäure zusammensetzung von AT III führen. Methoden zur gezielten Mutagenese, die mit Einzelstrang DNA bzw. Heteroduplex DNA arbeiten, sind bekannt (Morinaga et al., BioTechnology, 1984, 7, 636-639; Kramer et al., Nucl. Acid Res., 198, 12, 9441-9456). Tab. 2 zeigt beispielhaft einige Oligonukleotide, wie sie zur gezielten Mutagenese von humanem AT III eingesetzt wurden.

Es wurden nun Mutanten hergestellt, die an einer oder mehr der Glykosylierungsstellen Asn 96, Asn 135, Asn 155, Asn 192 eine andere Aminosäure, vorzugsweise Gln, tragen, was die Heaparinbindungs - /Heparinaktivierungseigenschaften unter Beibehaltung der Proteasespezifität verbessert; ferner wurden Mutanten hergestellt, die an Position Arg 393 (vorzugsweise Mutation zu Met oder Val) verändert sind, was eine Veränderung der Enzymspezifität bewirkt.

Mutanten mit verbesserten Heparinbindungs- / Heparinaktivierungseigenschaften besitzen Vorteile bei der AT III/Heparin-Kotherapie, weil gegebenenfalls mit niedrigeren Heparindosen therapiert werden kann.

Spezifitätsmutanten hingegen führen zu neuen Molekülen, bei denen die Eigenschaft der Heparinaktivierbarkeit von AT III auf mutierte Moleküle mit Affinität zu neuen Proteasen (z. B. Elastase, Plasmin etc.) übertragen werden kann, so daß solche Moleküle vorteilhafte, geänderte Therapiekonzepte aufgrund geänderter Dosierungen gestatten.

Mutierte AT III Proteine können in Säugerzellen exprimiert, über Standardmethoden gereinigt und bezüglich ihrer Proteasespezifität bzw. ihrer Heparinaktiverungseigenschaften, ihrer biochemisch/biophysikalischen Eigenschaften und/oder bezüglich klinischer Parameter untersucht werden. Die Synthese von veränderten Formen von AT III wird durch ein Vektor-/Wirt-Zellsystem, das schnell zu hohen Expressionsraten führt, erreicht (Zettlmeißl et al. (1988) Behring Inst. Mitt. 82, 26-34).

Es wurde gefunden, daß bei zwei oder mehr Mutationen an den Positionen Asn 96, Asn 135, Asn 155, Asn 192, Arg 393, wobei Arg 393 nicht gegen His oder Cys ausgetauscht ist, wertvolle AT III Mutanten mit verbesserten Heparinbindungs- /Heparinaktivierungseigenschaften erhalten werden.

Die Erfindung betriffl folglich Antithrombin III (AT III)-Mutanten mit verbesserter Heparinbindungs-/Heparinaktivierungseigenschaft, ausgewählt aus der Gruppe, die an den Positionen Asn 96, Asn 135, Asn 155, Asn 192, Arg 393 die natürlicherweise vorhandene Aminosäure ausgetauscht enthalten, wobei Arg 393 nicht gegen His oder Cys ausgetauscht ist

### Beispiel 1: Synthese von AT III-Mutanten (Allgemeines Verfahren)

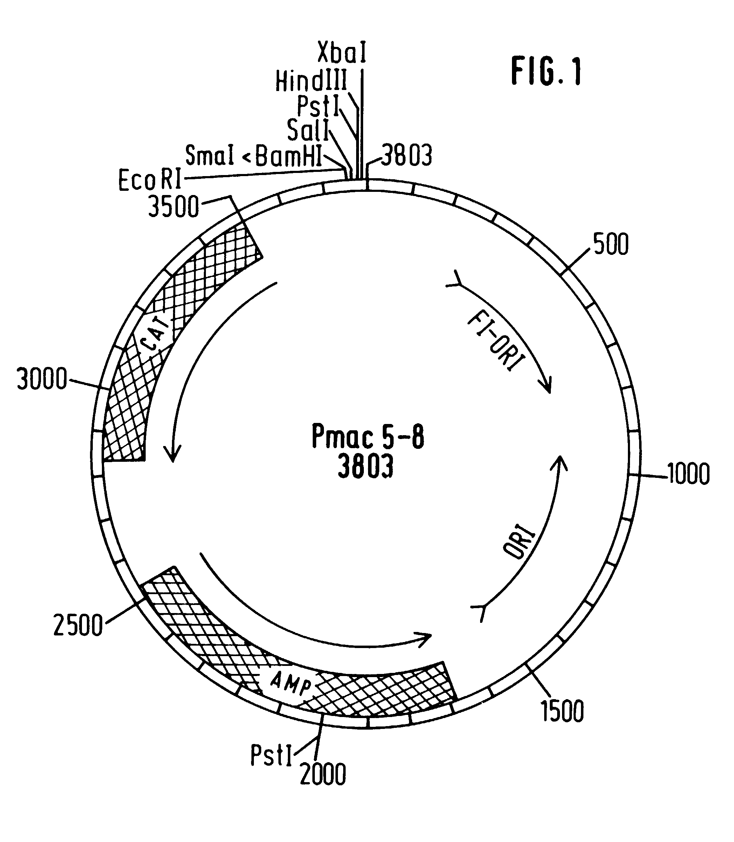

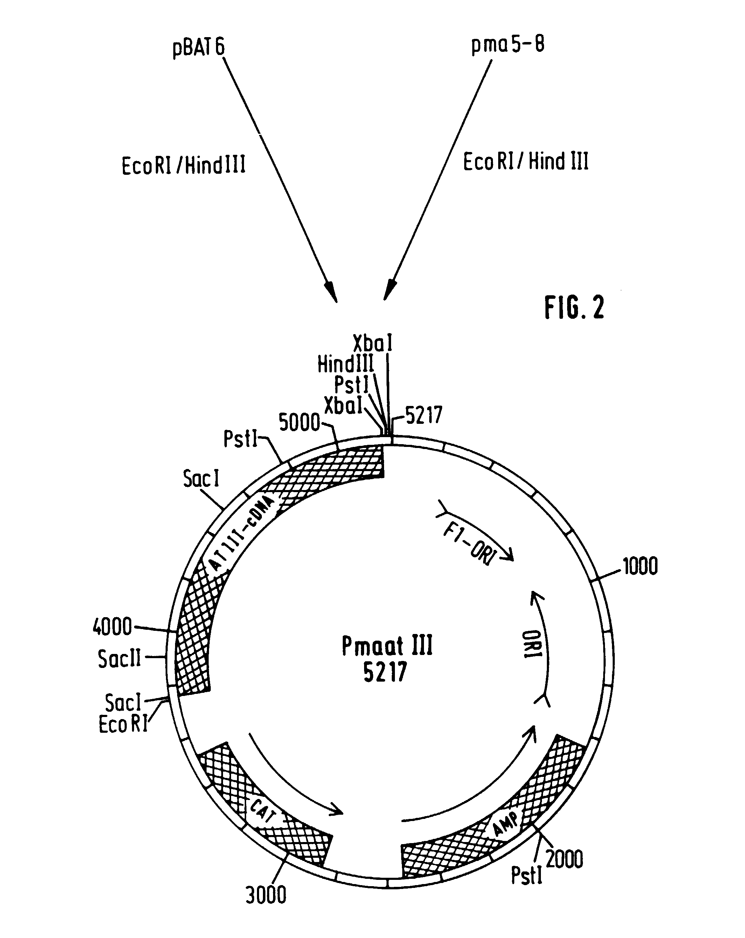

Aus dem Plasmid pbetaAT6 (EP 0 256 302 A2) wurde durch Verdauung mit EcoRI/HindIII ein 1,4 kb Fragment isoliert, das den gesamten kodierenden Bereich der humanen AT III cDNA enthält. Dieses Fragment wurde in den Polylinker (EcoRI/HindIII-gespalten) des Mutagenesevektors pMA 5-8 (Fig. 1) kloniert. Das resultierende Plasmid wurde pMAATIII genannt (Fig. 2).

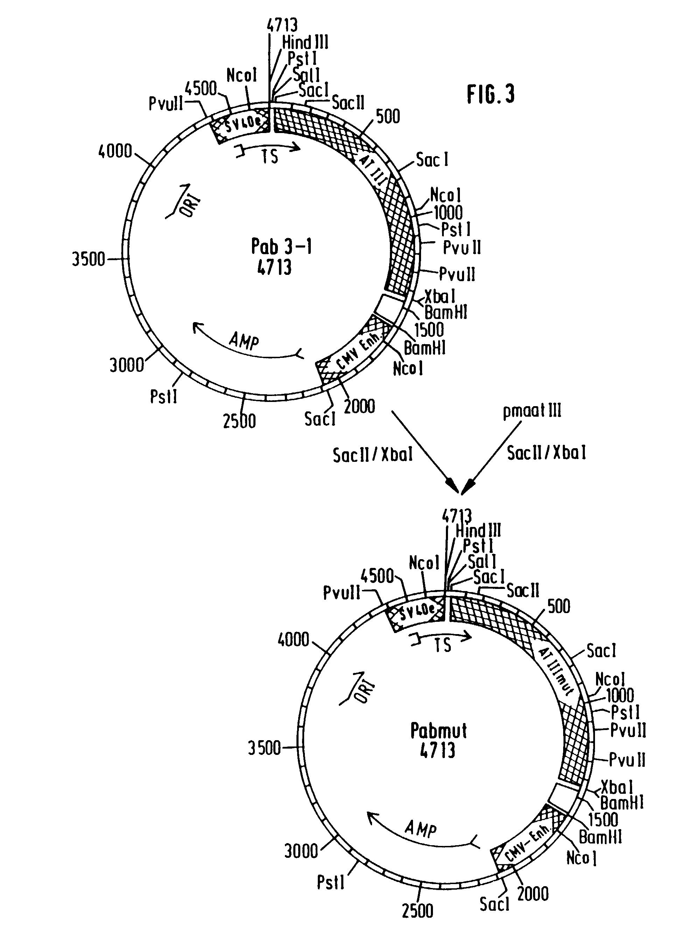

Nach Durchführung der Mutagenese (s.u.: Beschreibung "Mutagenese") wurde die mutierte cDNA durch Schneiden mit SacII/XbaI isoliert und in den Expressionsvektor pAB 3-1 (AT III Wildtyp), der ebenfalls mit SacII/XbaI verdaut wurde, kloniert, was zu dem Plasmid pABmut führte (Fig. 3). Die pABmut-Plasmide tragen neben der jeweiligen mutierten cDNA die SV40 "early" Enhancer/Promotor-Einheit, die SV40 Polyadenylierungsstelle für frühe Transkripte und den CMV "immediate early" Enhancer (Zettlmeißl et al. a.a.O).

Die pABmut-Plasmide wurden über CsCl-Gradienten gereinigt und, wie bei Zettlmeißl et al., a.a.O. beschrieben, mit den Plasmiden pSV2dhfr und pRMH140 in BHK-Zellen (ATCC CCL10) kotransfiziert. Nach Doppelselektion in DME-Medium + 10% FKS + 400 µg/ml G418 und 1 µM Methotrexat (Standardwachstumsmedium) wurden die auftretenden resistenten Klone (ca. 40 - 100) als Klonmischung in T25-Kulturgefäßen vereinigt. Die Mischklone wurden über T80 und T180 Kulturgefäße in Standardwachstumsmedium auf Plastikrollerflaschen (1750 cm²) expandiert und dort adhärent zur Konfluenz gezüchtet. Die konfluenten Zellen wurden zweimal mit 200 ml Iscove's Medium (Behringwerke AG, Marburg) gewaschen (je 2 Stunden bei 37°C) und anschliessend mit 500 ml desselben Mediums als Erntemedium 48 Stunden lang gerollt. Das Erntemedium wurde durch Zentrifugation von zellulären Bestandteilen befreit. Aliquots der konditionierten Erntemedien wurden in einem für humanes AT III spezifischen ELISA (Zettlmeißl et al. 1987, BioTechnology 5, 720-725) bezüglich ihres AT III-Antigengehalts untersucht. Hierbei ermittelte Expressionswerte für Wildtyp AT III (AT III-WT) und verschiedene Mutanten sind in Tab. 3 exemplarisch angegeben.

AT III-WT und davon abgeleitete mutierte Proteine wurden aus den Erntemedien nach einem Standardverfahren (Affinitätschromatographie mit Heparin-Sepharose, gefolgt von fraktionierter Ammoniumsulfatfällung) aufgereinigt (Zettlmeißl et al. 1987) und anschließend charakterisiert.

Mutante AT III Moleküle können auch mit anderen Expressionsvektoren in verschiedenen permanenten Säugerzellinien entsprechend dem Stand der Technik exprimiert werden.

### Beispiel 2: Mutagenese / Allgemeines Verfahren (Kramer et al., Nucl. Acids Res. (1984) 12, 9441-9456)

Einzelstrang DNA des Mutagenesevektors pMAATIII (Fig. 2), welcher in dem E.coli Stamm WK6 transformiert worden war, wurde nach Standardmethoden isoliert.

Plasmid DNA von pMC5-8 (siehe Legende zu Fig. 1) wurde mit EcoRI/HindIII geschnitten und das Vektorfragment (3.8kb) aus einem Agarosegel über Papierelution (Maniatis et al. 1982, Molecular Cloning - A Laboratory Manual, Cold Spring Harbor, New York) gereinigt.

Zur Herstellung einer "gapped-duplex" DNA wurden 0,1 pmol doppelsträngiges Fragment (aus pMC) und 0,5 pmol Einzelstrang DNA (pMAATIII) in 12,5 mM Tris-HCl pH 7,5 + 190 mM KCl (Endvolumen 40 µl) für 4 Minuten bei 100°C erhitzt und anschließend 10 Minuten bei 65°C inkubiert. Zur Anhybridisierung des Mutageneseoligonukleotids (s. Tab. 2) wurden 8 µl der genannten Hybridisierungslösung mit 4 - 8 pmol (2 µl) des enzymatisch phosphorylierten Oligonukleotids für 5 Minuten auf 65°C erhitzt und dann langsam auf Raumtemperatur abgekühlt. Nach Zugabe von 24 µl H₂O, 4 µl 10fachem "fill-in" Puffer (625 mM KCl, 275 mM Tris-HCl pH 7,5, 150 mM MgCl₂, 20 mM DTT, 0,5 mM ATP und je 0,25 mM der vier dNTP's), 1 µl T4-DNA-Ligase (5 U/µl) und 1 µl Klenow-Fragment der DNA Polymerase I (1 U/µl) folgte eine 45minütige Inkubation bei Raumtemperatur. 5 µl aufgefüllter gapped-duplex DNA wurden in WK6 muts (mutS215:Tn10) transformiert. Der gesamte Transformationsansatz wird in einer Schüttelkultur in LB-Medium + 25 µg/ml Chloramphenicol (10 ml) über Nacht bei 37°C vermehrt. Aus dem gesamten Ansatz wurde die Plasmid-DNA nach Standardmethoden gereinigt (Maniatis et al. 1982). Etwa 20 ng des gereinigten Plasmids wurden in WK6 transformiert. Die Transformanten wurden auf LB-Platten mit 25 µg/ml Chloramphenicol selektioniert.

Fünf dieser Transformanten wurden durch eine geeignete Sequenzreakticn (C-, T-, A- oder G-spezifisch) auf die gewünschte Mutation hin analysiert. Positive Klone wurden durch detaillierte Sequenzanalyse im Bereich der Mutagenesestelle bestätigt (Sanger et al. (1977), Proc.Natl. Acad.Sci. USA 74, 5463-5467).

### Beispiel 3: AT III - Met 393; AT III - Val 393 und AT III - Leu 393

Mit dem Ziel, ein Molekül mit Alphal-Antitrypsin ähnlicher Spezifität (Elastaseinhibitor) zu erzeugen, wurde Arg393 von AT III (P1-Position) in ein Met, Val bzw. Leu umgewandelt.: Zur Mutagenese wurden die Oligonukleotide Nr. 1, 2 und 3 aus Tab. 2 eingesetzt.

Die Mutanten werden in vergleichbaren Mengen wie AT III-Wildtyp (WT) von BHK-Zellen synthetisiert und ins Kulturmedium ausgeschleußt (Tab.4) , zeigen im Vergleich zu AT III-Plasma und AT III-WT identisches Verhalten gegenüber anti-AT III-Seren aus Kaninchen und lassen sich in Analogie zum AT III-WT nach dem oben beschriebenen Standardverfahren zu Reinheiten größer 95% aufreinigen. Das Bindungs- und Elutionsverhalten dieser Mutanten auf Heparinsepharose zeigt keinen Unterschied zu AT III-WT. Dies weist auf intaktes Heparinbindungs- und Heparinaktivierungsverhalten der Mutante hin.

Die Mutanten zeigen keine Progressivinhibitor- (Hensen et al., 1963, Thromb. Diatl. Haemorrh. 9, 18-29) und Heparinkofaktoraktivität (Schrader et al., 1986, Ärztl. Lab. 32, 111-114) gegenüber Thrombin mehr (Tab. 3).

Die drei Mutanten inhibieren im Gegensatz zu AT III-WT Leukozyten-Elastase. Die Elastase wurde aus menschlichen Leukozyten nach der von Engelbrecht et al. (Hoppe-Seyler's Ztschr. Physiol. Chemie 363, 305-315, 1982) beschriebenen Methode isoliert. Als Substrat wurde das von Nakajima et al. (J. Biol. Chem. 254, 4027-4032, 1979) beschriebene MeO-Suc-Ala-Ala-Pro-Val-pNA (Calbiochem) benutzt. Die Freisetzung des Paranitroanilins aus dem Substrat wurde als Zunahme der Absorption bei 405 nm im Spektralphotometer innerhalb von 15 min. gemessen. Dieser Absorptionswert wurde als 100 % Enzymaktivität der PMN Elastase definiert. Die Inhibitoren wurden in steigenden Konzentrationen bis maximal 100 µg/ml für eine Stunde mit dem Enzym vorinkubiert. Die Enzymreaktion wurde dann mit dem Substrat gestartet. Der Test wurde in 0.1 mol/l HEPES, pH 7.5, + 0.1 mol/l Natriumchlorid durchgeführt. Die Substratkonzentration betrug 0.13 mmol/l. Als IC50 in µg/ml wurde diejenige Inhibitorkonzentration bezeichnet, die 50% der Enzymaktivität inhibierte. Substanzen, die bei einer Maximalkonzentration von 100 µg/ml keine inhibierende Wirkung zeigten, wurden als wirkungslos definiert. Als Referenzinhibitor diente alpha1-Proteaseinhibitor (alpha1-PI, alpha1-Antitrypsin), der einen Inhibitionswert von 3.7 hatte. AT III-WT zeigte keine Hemmung der PMN Elastaseaktivität. AT III Val zeigte eine dem alphal-PI vergleichbare Aktivität von 4.0 µg/ml, während AT III Met und AT III Leu mit 28 bzw. 65 µg/ml deutlich weniger aktiv waren (s. nachfolgende Tabelle).

Mit dem beschriebenen PMN-Elastase Test wurde für AT III - Val 393 im Vergleich zu alphal PI die KI-Bestimmung durchgeführt. Die eingesetzten Konzentrationen des Substrates MeO-Suc-Ala-Ala-Pro-Val-pNA betrugen 0.0011, 0.0022, 0.0044, 0.0087, 0.0175, 0.035, 0.7 mmol/l. Die Konzentration des Inhibitors betrug 3,5 x 10⁻⁸ 8 mol/l.

In beiden Fällen handelt es sich bei der Hemmung der PMN-Elastase um eine nicht kompetitive Hemmung (s. nachfolgende Tabelle). Die KI-Werte für alphal PI und AT III - Val 393 sind nahezu identisch.

### Beispiel 4: AT III-Gln135 und AT III-Gln155

Ein Ziel der in dieser Anmeldung als Erfindung beanspruchten Experimente ist es, den Einfluß der Glykosylierung im AT III Molekül auf die biologischen und biochemischen Eigenschaften von AT III zu untersuchen.

Durch Asn→Gln Austausche in den Positionen 135 bzw. 155 wurden zwei AT III-Mutanten (AT III-Gln135 und AT III-Gln155) erzeugt, denen jeweils eine Kohlehydratseitenkette fehlt. Als Mutageneseoligonukleotide wurden die Oligonukleotide 8 und 9 (Tab. 2) eingesetzt. Die Expressionsraten für beide Mutanten in BHK-Zellen (AT III im Kulturmedium) liegen, wie in Tab. 4 gezeigt, in derselben Größenordnung wie für AT III-WT. Beide Mutanten zeigen im Vergleich zu AT III-Plasma und AT III-WT identisches Verhalten gegenüber anti-AT III-Seren aus Kaninchen, bezüglich spezifischer Progressivinhibitor- und Heparinkofaktoraktivität (Tab. 4) und lassen sich nach dem oben beschriebenen Standardverfahren zu Reinheiten größer 95% aufreinigen.

Beide Mutanten wurden bezüglich ihrer relativen Thrombininaktivierungsfähigkeit in Abhängigkeit von der Heparinkonzentration im Test untersucht und mit AT III-Plasma, AT III-WT bzw. der Mutante AT III-Lys49 verglichen (Tab. 5).

Der Test wurde unter folgenden Bedingungen durchgeführt: 0,02 U (Antigen)/ml AT III (AT III-Plasma, AT III-WT oder AT III-Mut) wurden mit 0,3 IU/ml alpha-Thrombin (human), 2 KIU/ml Aprotinin (Behringwerke) und Heparin (Hoffmann-LaRoche) in Konzentrationen von 0 - 25 IU/ml wurden in einem Volumen von 1 ml 5 Minuten bei 37°C vorinkubiert. Nach Zugabe von 100 µl Substratreagenz (2 mM HD-CHA-But-Arg-pNA) wurde die Extinktionsänderung bei 405 nm (37°C) kinetisch verfolgt. Maximale Inhibition von alpha-Thrombin bei Heparinsättigung wurde 100% gesetzt.

Die Mutanten AT III-Gln135 und AT III-Glnl55 zeigen im Vergleich zu AT III-Plasma und AT III-WT eine verbesserte Inhibition von Thrombin bei niedrigen Heparinkonzentrationen (Tab. 5).

Tab. 5 gibt die Heparinkonzentration bei halbmaximaler Thrombininhibition (c 1/2) für AT III-Plasma, AT III-WT und verschiedene AT III-Mutanten an.

### Beispiel 5: AT III-Gln135/155

Die in Beispiel 4 beschriebenen Mutationen wurden durch sequentielle Mutagenese mit den Oligonukleotiden 8 und 9 (Tab. 2) in einem AT III-Molekül vereinigt. Das mutierte Protein verhält sich im beschriebenen Standardreinigungsverfahren wie ein rekombinantes Wildtyp AT III-Molekül.

Die bei AT III-Gln135 und bei AT III-Gln155 verbesserte Inhibition von alpha-Thrombin bei niedrigen Heparinkonzentrationen ist im Falle von AT III-Gln135/155 noch stärker ausgeprägt (Tab. 5).

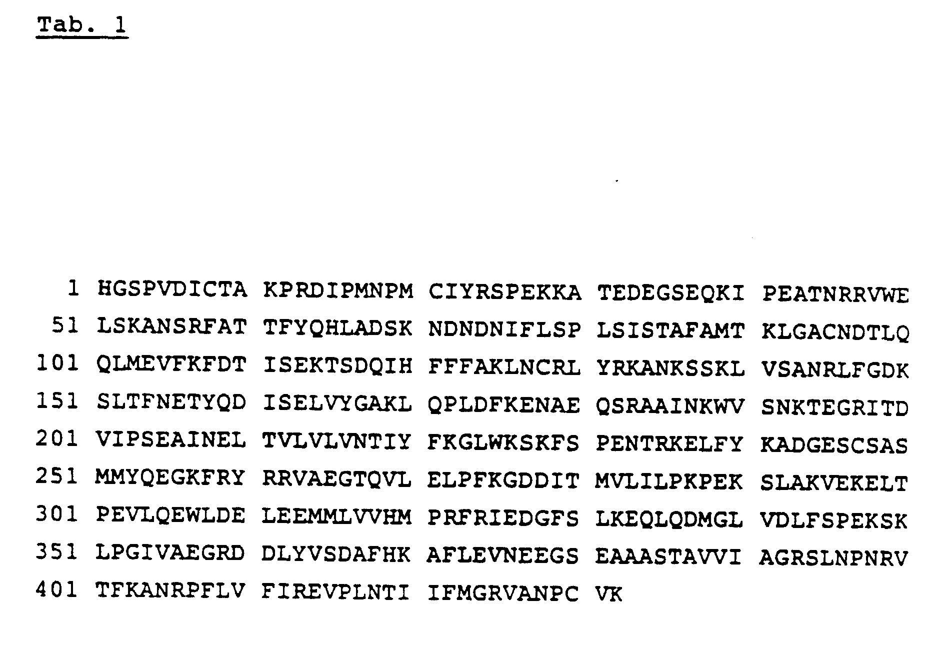

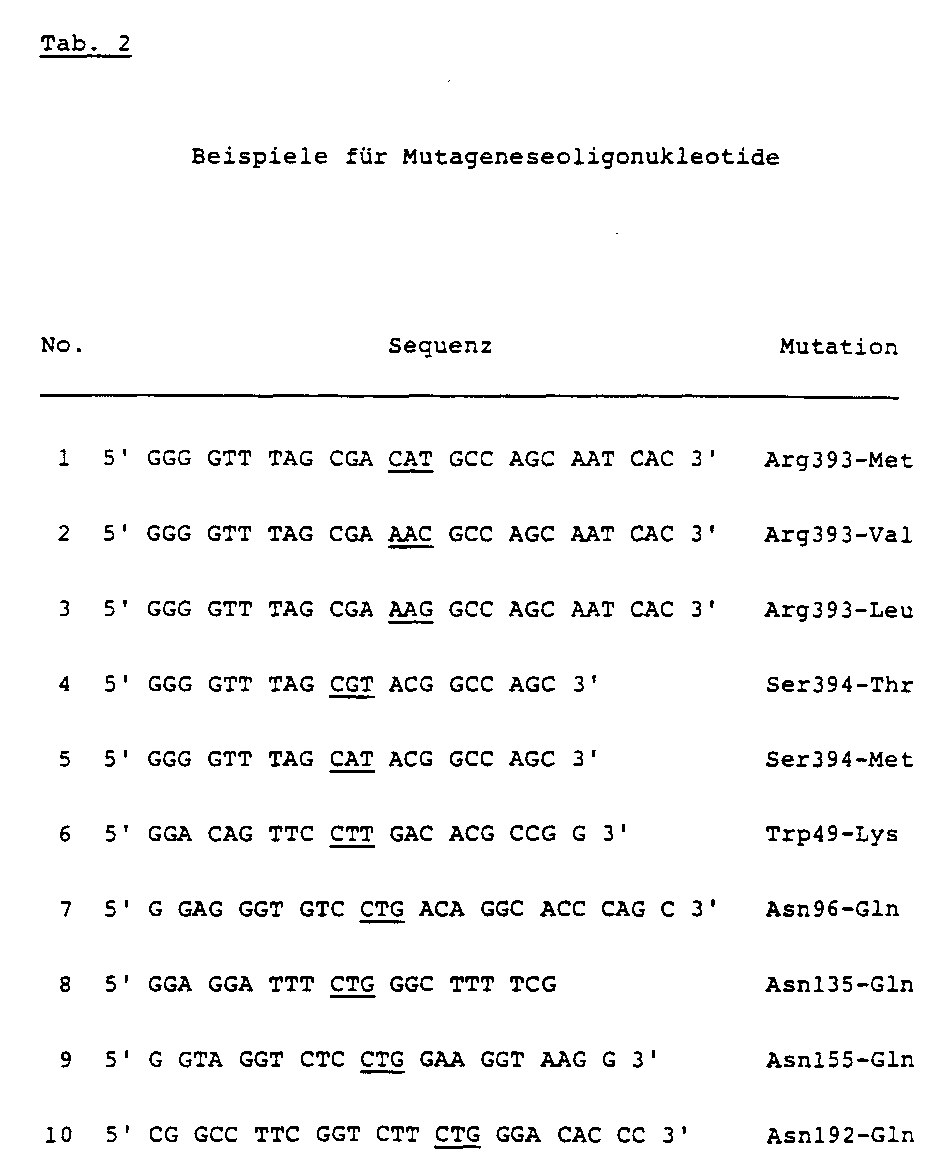

**Tab. 3**

| Inhibition der Elastase aus humanen polymorphkernigen Granulozyten (PMN Elastase) | | |
|---|---|---|
| Substanz | IC₅₀ (µg/ml) | k_{I} (mol/l) |
| alpha₁ PI | 3.7 | 1,7 x 10⁻⁸ |
| AT III - WT | - | n.b. |
| AT III - Met 393 | 28.0 | n.b. |
| AT III - Val 393 | 4.0 | 1 x 10⁻⁸ |
| AT III - Leu 393 | 65.0 | n.b. |
| - = keine Inhibition (IC₅₀ > 100 µg/ml) n.b. = nicht bestimmt | | |

**Tab. 4**

| Expression und Reinigung von AT III-Mutanten | | | | |
|---|---|---|---|---|
| | konz.in Rollerüberständen ¹⁾(mg/l) | Reinigung nach Standardverf. | PI²⁾ (U/mg) | HC³⁾(U/mg) |
| ATIII-Plasma | - | +++ | 4-6,5* | 4-6,5* |
| ATIII-WT | 4,2 | +++ | 6,2 | 5 |
| ATIII-Met393 | 5,5 | +++ | 0 | 0 |
| ATIII-Val393 | 4,8 | +++ | 0 | 0 |
| ATIII-Leu393 | 9,8 | +++ | 0 | 0 |
| ATIII-Thr394 | 9,7 | +++ | n.b. | 3,5 |
| ATIII-Lys49 | 3,3 | ++ | 4,6 | 4,3 |
| ATIII-Glnl35 | 8 | +++ | 3,9 | 4,5 |
| ATIII-Glnl55 | 3,6 | +++ | 4,2 | 5,5 |
| ATIII-Gln135/155 | 1,2 | +++ | n.b. | 3,8 |

| | | | | |
|---|---|---|---|---|
| ¹⁾ 48 h serumfreie Überstände (ELISA) von BHK-Zellen | | | | |
| ²⁾ Progressivinhibitoraktivität (Hensen et al. 1963) | | | | |
| ³⁾ Heparinkofaktoraktivität (Schrader et al. 1986) n.b. = nicht bestimmt | | | | |
| * = Chargenabhängige Schwankungsbreite | | | | |

**Tab. 5**

| Abhängigkeit der Thrombininaktivierung von der Heparinkonzentration | |
|---|---|
| | Heparin ¹⁾ Cl/2 (mIU/ml) |
| ATIII-Plasma | 65 |
| ATIII-WT | 65 |
| ATIII-Gln135 | 22 |
| ATIII-Gln155 | 22 |
| ATIII-Gln135/155 | 5 |
| ATIII-Lys49 | größer 360 |

| | |
|---|---|
| ¹⁾ Heparinkonzentration bei halbmaximaler relativer Thrombininhibition | |

### Legende zu Fig. 1:

Karte der Plasmide pMAC5-8 (= pMA5-8 und pMC5-8).

| | |
|---|---|
| F1-ORI | Replikationsursprung des Phagen fl; |
| ORI | Replikationsursprung vom ColE1-Typ; |
| CA | Kodierende Region für Chloramphenicol-acetyl transferase; |
| AMP | Kodierende Region für β-Lactamase. |

pMA5-8 trägt eine amber-Mutation in CAT (A bei Position 3409) und pMC5-8 eine amber-Mutation in AMP (C bei Position 2238).

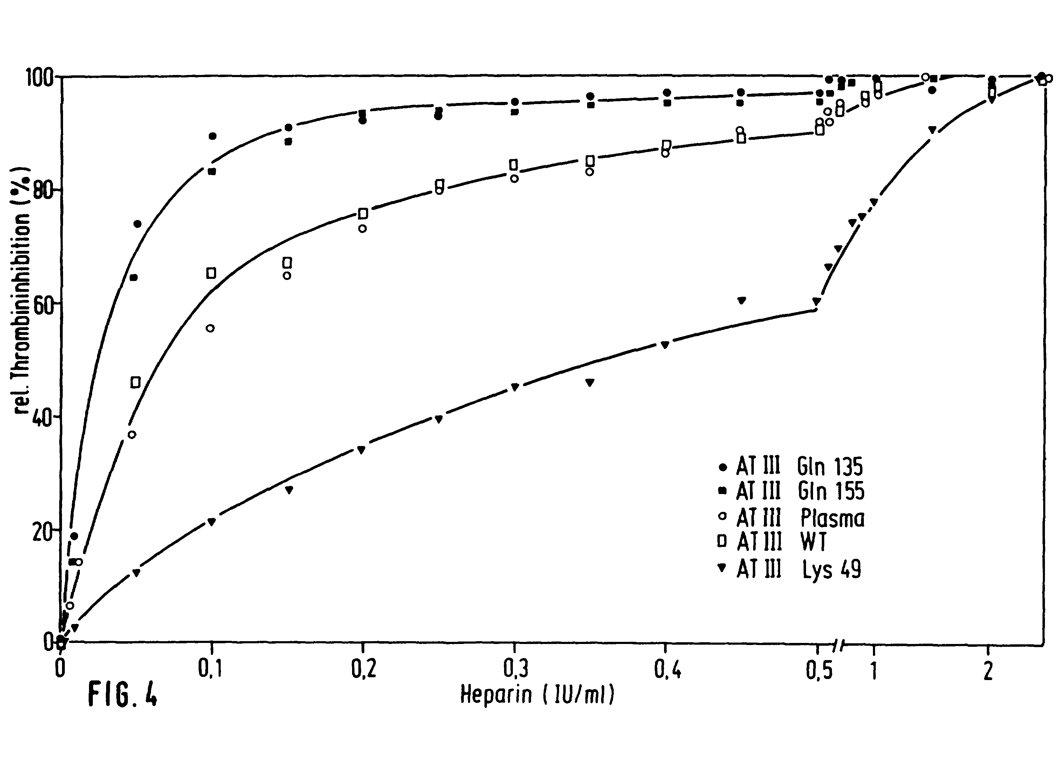

### Legende zu Fig. 4:

Abhängigkeit der relativen Inhibition von humanem alpha-Thrombin durch AT III-Plasma (o), AT III-WT (□), AT III-Gln135 (●), AT III-Glnl55 (■) und AT III-Lys (▼). Die experimentellen Bedingungen sind in Beispiel 4 beschrieben.

## Patentansprüche (Patentansprüche für folgende(n) Vertragsstaat(en): AT, BE, CH, LI, DE, DK, FR, GB, IT, LU, NL, SE)

1. Antithrombin III (ATIII)-Mutanten mit verbesserter Heparinbindungs- / Heparinaktivierungseigenschaft, ausgewählt aus der Gruppe, die an den Positionen Asn96, Asn135, Asn155, Asn192, Arg393 die natürlicherweise vorhandene Aminosäure an mindestens zwei Positionen gegen eine andere Aminosäure ausgetauscht enthalten, wobei Arg393 nicht gegen His oder Cys ausgetauscht ist.

2. AT III-Mutanten nach Anspruch 1, die an mindestens zwei Positionen mutiert sind, wobei die Mutationen aus der Gruppe Arg393 ⇒ Met, Arg393 ⇒ Val. Arg393 ⇒ Leu, Asn96 ⇒ Gln, Asn135 ⇒ Gln, Asn155 ⇒ Gln, Asn192 ⇒ Gln ausgewählt sind.

3. ATIII-Mutanten nach Anspruch 2, die die Mutation Arg393 ⇒ Met, Arg393 ⇒ Val, oder Arg393 ⇒ Leu, zusammen mit einer oder mehreren der Mutationen Asn96 ⇒ Gln, Asn135 ⇒ Gln, Asn155 ⇒ Gln, Asn192 ⇒ Gln enthalten.

4. ATIII-Mutanten nach Anspruch 2, die zwei oder mehrere der Mutationen Asn96 ⇒ Gln, Asn135 ⇒ Gln, Asn155 ⇒ Gln, Asn192 ⇒ Gln enthalten.

5. ATIII-Mutante nach Anspruch 2, die die Mutation Arg393 ⇒ Met enthält.

6. ATIII-Mutante nach Anspruch 2, die die Mutation Arg393 ⇒ Val enthält.

7. ATIII-Mutante nach Anspruch 2, die die Mutation Arg393 ⇒ Leu enthält.

8. ATIII-Mutante nach Anspruch 2, die die Mutation Asn96 ⇒ Gln enthält.

9. ATIII-Mutante nach Anspruch 2, die die Mutation Asn135 ⇒ Gln enthält.

10. ATIII-Mutante nach Anspruch 2, die die Mutation Asn155 ⇒ Gln enthält.

11. ATIII-Mutante nach Anspruch 2, die die Mutation Asn192 ⇒ Gln enthält.

12. Verfahren zur Herstellung einer der Mutanten nach Anspruch 1 bis 11, dadurch gekennzeichnet, daß die dafür kodierende cDNA in geeigneten pro- oder eukaryotischen Expressionssystemen exprimiert wird.

13. Arzneimittel, das eine oder mehrere der Mutanten nach Anspruch 1 bis 11 enthält.

## Patentansprüche (Patentansprüche für folgende(n) Vertragsstaat(en): ES, GR)

1. Verfahren zur Herstellung von Antithrombin III (AT (ATIII)-Mutanten, mit verbesserter Heparinbindungs- / Heparinaktivierungseigenschaft, ausgewählt aus der Gruppe, die an den Positionen Asn 96, Asn 135, Asn 155, Asn 192, Arg 393 die natürlicherweise vorhandene Aminosäure an mindestens zwei Positionen gegen eine andere Aminosäure ausgetauscht enthalten, wobei Arg 393 nicht gegen His oder Cys ausgetauscht ist, dadurch gekennzeichnet, daß die jeweils für die genannten Mutanten kodierende cDNA in geeigneten pro- oder eukaryontischen Expressionssystemen exprimiert wird.

2. Verfahren nach Anspruch 1, wobei die Mutationen aus der Gruppe Arg 393 ⇒ Met, Arg 393 ⇒ Val, Arg 393 ⇒ Leu, Asn 96 ⇒ Gln, Asn 135 ⇒ Gln, Asn 155 ⇒ Gln, Asn 192 ⇒ Gln ausgewählt sind.

3. Verfahren nach Anspruch 2, wobei die Mutation Arg 393 ⇒ Met, Arg 393 ⇒ Val, oder Arg 393 ⇒ Leu, zusammen mit einer oder mehreren der Mutationen Asn 96 ⇒ Gln, Asn 135 ⇒ Gln, Asn 155 ⇒ Gln, Asn 192 ⇒ Gln enthalten sind.

4. Verfahren nach Anspruch 2, wobei zwei oder mehrere der Mutationen Asn 96 ⇒ Gln, Asn 135 ⇒ Gln, Asn 155 ⇒ Gln, Asn 192 ⇒ Gln enthalten sind.

5. Verfahren nach Anspruch 2, wobei die Mutation Arg 393 ⇒ Met enthalten ist.

6. Verfahren nach Anspruch 2, wobei die Mutation Arg 393 ⇒ Val enthalten ist.

7. Verfahren nach Anspruch 2, wobei die Mutation Arg 393 ⇒ Leu enthalten ist.

8. Verfahren nach Anspruch 2, wobei die Mutation Asn 96 ⇒ Gln enthalten ist.

9. Verfahren nach Anspruch 2, wobei die Mutation Asn 135 ⇒ Gln enthalten ist.

10. Verfahren nach Anspruch 2, wobei die Mutation Asn 155 ⇒ Gln enthalten ist.

11. Verfahren nach Anspruch 2, wobei die Mutation Asn 192 ⇒ Gln enthalten ist.

## Claims (Claims for the following Contracting State(s): AT, BE, CH, LI, DE, DK, FR, GB, IT, LU, NL, SE)

1. An antithrombin III (AT III) mutant with improved heparin-binding/heparin-activating property selected from the group which comprises the amino acid naturally present at the positions Asn96, Asn135, Asn155, Asn192, Arg393 being replaced at a minimum of two positions by another amino acid, with Arg393 not being replaced by His or Cys.

2. An AT III mutant as claimed in claim 1 which is mutated at a minimum of two positions, the mutations being selected from the group of Arg393 ⇒ Met, Arg393 ⇒ Val, Arg393 ⇒ Leu, Asn96 ⇒ Gln, Asn135 ⇒ Gln, Asn155 ⇒ Gln, Asn192 ⇒ Gln.

3. An ATIII mutant as claimed in claim 2 which comprises the mutation Arg393 ⇒ Met, Arg393 ⇒ Val, or Arg393 ⇒ Leu, together with one or more of the mutations Asn96 ⇒ Gln, Asn135 ⇒ Gln, Asnl55 ⇒ Gln, Asn192 ⇒ Gln.

4. ATIII mutants as claimed in claim 2, which contain two or more of the mutations Asn96 ⇒ Gln, Asn135 ⇒ Gln, Asn155 ⇒ Gln, Asn192 ⇒ Gln.

5. An ATIII mutant as claimed in claim 2, which contains the mutation Arg393 ⇒ Met.

6. An ATIII mutant as claimed in claim 2, which contains the mutation Arg393 ⇒ Val.

7. An ATIII mutant as claimed in claim 2, which contains the mutation Arg393 ⇒ Leu.

8. An ATIII mutant as claimed in claim 2, which contains the mutation Asn96 ⇒ Gln.

9. An ATIII mutant as claimed in claim 2, which contains the mutation Asn135 ⇒ Gln.

10. An ATIII mutant as claimed in claim 2, which contains the mutation Asn155 ⇒ Gln.

11. An ATIII mutant as claimed in claim 2, which contains the mutation Asn192 ⇒ Gln.

12. A process for preparing one of the mutants as claimed in claims 1 to 11, which comprises expressing the cDNA coding therefor in suitable pro- or eukaryotic expression systems.

13. A pharmaceutical which contains one or more of the mutants as claimed in claim 1 to 11.

## Claims (Claims for the following Contracting State(s): ES, GR)

1. A process for preparing antithrombin III (AT III) mutants with improved heparin-binding/heparin-activating property selected from the group which comprises the amino acid naturally present at the positions Asn 96, Asn 135, Asn 155, Asn 192, Arg 393 being replaced at a minimum of two positions by another amino acid, with Arg 393 not being replaced by His or Cys, wherein the cDNA coding for said mutants in each case is expressed in suitable pro- or eukaryotic expression systems.

2. The process as claimed in claim 1, where the mutations are selected from the group of Arg 393 ⇒ Met, Arg 393 ⇒ Val, Arg 393 ⇒ Leu, Asn 96 ⇒ Gln, Asn 135 ⇒ Gln, Asn 155 ⇒ Gln, Asn 192 ⇒ Gln.

3. The process as claimed in claim 2, where the mutation Arg 393 ⇒ Met, Arg 393 ⇒ Val, or Arg 393 ⇒ Leu is present together with one or more of the mutations Asn 96 ⇒ Gln, Asn 135 ⇒ Gln, Asn 155 ⇒ Gln, Asn 192 ⇒ Gln.

4. The process as claimed in claim 2, two or more of the mutations Asn 96 ⇒ Gln, Asn 135 ⇒ Gln, Asn 155 ⇒ Gln, Asn 192 ⇒ Gln being contained.

5. The process as claimed in claim 2, the mutation Arg 393 ⇒ Met being contained.

6. The process as claimed in claim 2, the mutation Arg 393 ⇒ Val being contained.

7. The process as claimed in claim 2, the mutation Arg 393 ⇒ Leu being contained.

8. The process as claimed in claim 2, the mutation Asn 96 ⇒ Gln being contained.

9. The process as claimed in claim 2, the mutation Asn 135 ⇒ Gln being contained.

10. The process as claimed in claim 2, the mutation Asn 155 ⇒ Gln being contained.

11. The process as claimed in claim 2, the mutation Asn 192 ⇒ Gln being contained.

## Revendications (Revendications pour l'(les) Etat(s) contractant(s) suivant(s): AT, BE, CH, LI, DE, DK, FR, GB, IT, LU, NL, SE)

1. Mutants de l'antithrombine III (AT III) à propriété améliorée de liaison à l'héparine / activation par l'héparine, choisis parmi ceux qui comportent aux positions Asn96, Asn135, Asn155, Asn192, Arg393 l'aminoacide naturellement présent remplacé en au moins deux positions par un autre aminoacide, Arg393 n'étant pas remplacé par His ou Cys.

2. Mutants d'AT III selon la revendication 1, qui sont mutés en au moins deux positions, les mutations étant choisies parmi Arg393 ⇒ Met, Arg393 ⇒ Val, Arg393 ⇒ Leu, Asn96 ⇒ Gln, Asn135 ⇒ Gln, Asn155 ⇒ Gln, Asn192 ⇒ Gln.

3. Mutants d'AT III selon la revendication 2, qui comportent la mutation Arg393 ⇒ Met, Arg393 ⇒ Val ou Arg393 ⇒ Leu, conjointement avec une ou plusieurs des mutations Asn96 ⇒ Gln, Asn135 ⇒ Gln, Asn155 ⇒ Gln, Asn192 ⇒ Gln.

4. Mutants d'AT III selon la revendication 2, qui comportent deux ou plus de deux des mutations Asn96 ⇒ Gln, Asn135 ⇒ Gln, Asn155 ⇒ Gln, Asn192 ⇒ Gln.

5. Mutant d'AT III selon la revendication 2, qui comporte la mutation Arg393 ⇒ Met.

6. Mutant d'AT III selon la revendication 2, qui comporte la mutation Arg393 ⇒ Val.

7. Mutant d'AT III selon la revendication 2, qui comporte la mutation Arg393 ⇒ Leu.

8. Mutant d'AT III selon la revendication 2, qui comporte la mutation Asn96 ⇒ Gln.

9. Mutant d'AT III selon la revendication 2, qui comporte la mutation Asn135 ⇒ Gln.

10. Mutant d'AT III selon la revendication 2, qui comporte la mutation Asn155 ⇒ Gln.

11. Mutant d'AT III selon la revendication 2, qui comporte la mutation Asn192 ⇒ Gln.

12. Procédé pour la production d'un des mutants selon l'une des revendications 1 à 11, caractérisé en ce que l'ADNc codant pour celui-ci est exprimé dans des systèmes d'expression procaryotes ou eucaryotes appropriés.

13. Médicament, qui contient un ou plusieurs des mutants selon l'une des revendications 1 à 11.

## Revendications (Revendications pour l'(les) Etat(s) contractant(s) suivant(s): ES, GR)

1. Procédé pour la production de mutants de l'antithrombine III (AT III) à propriété améliorée de liaison à l'héparine / activation par l'héparine, choisis parmi ceux qui comportent aux positions Asn96, Asn135, Asnl55, Asn192, Arg393 l'aminoacide naturellement présent remplacé en au moins deux positions par un autre aminoacide, Arg393 n'étant pas remplacé par His ou Cys, caractérisé en ce que l'ADNc codant respectivement pour lesdits mutants est exprimé dans des systèmes d'expression procaryotes ou eucaryotes appropriés.

2. Procédé selon la revendication 1, dans lequel les mutations sont choisies parmi Arg393 ⇒ Met, Arg393 ⇒ Val, Arg393 ⇒ Leu, Asn96 ⇒ Gln, Asn135 ⇒ Gln, Asn155 ⇒ Gln, Asn192 ⇒ Gln.

3. Procédé selon la revendication 2, dans lequel sont contenues la mutation Arg393 ⇒ Met, Arg393 ⇒ Val ou Arg393 ⇒ Leu, conjointement avec une ou plusieurs des mutations Asn96 ⇒ Gln, Asn135 ⇒ Gln, Asn155 ⇒ Gln, Asn192 ⇒ Gln.

4. Procédé selon la revendication 2, dans lequel sont contenues deux ou plus de deux des mutations Asn96 ⇒ Gln, Asn135 ⇒ Gln, Asn155 ⇒ Gln, Asn192 ⇒ Gln.

5. Procédé selon la revendication 2, dans lequel la mutation Arg393 ⇒ Met est contenue.

6. Procédé selon la revendication 2, dans lequel la mutation Arg393 ⇒ Val est contenue.

7. Procédé selon la revendication 2, dans lequel la mutation Arg393 ⇒ Leu est contenue.

8. Procédé selon la revendication 2, dans lequel la mutation Asn96 ⇒ Gln est contenue.

9. Procédé selon la revendication 2, dans lequel la mutation Asn135 ⇒ Gln est contenue.

10. Procédé selon la revendication 2, dans lequel la mutation Asn155 ⇒ Gln est contenue.

11. Procédé selon la revendication 2, dans lequel la mutation Asn192 ⇒ Gln est contenue.
